# FASCICULE DE BREVET EUROPEEN

(11) **EP 3 025 056 B1**
(45) Date de publication et mention de la délivrance du brevet: **22.03.2017**
(21) Numéro de dépôt: 14738554.6
(22) Date de dépôt: 11.06.2014
(51) Int. Cl.: F04B 7/00, F04B 9/04, F04B 13/02, F04B 53/14, F16K 11/076, F16K 11/085, F04B 7/06

(54) **SOUS-ENSEMBLE OSCILLO-ROTATIF POUR POMPAGE D'UN FLUIDE ET DISPOSITIF DE POMPAGE OSCILLO-ROTATIF**
OSZILIERENDE UN ROTIERENDE UNTER-EINHEIT ZUM FÖRDERN EINES FLUIDS, SOWIE EINE OSZILIERENDE UN ROTIERENDE PUMPVORRICHTUNG
RECIPROCATING AND ROTATING SUB-GROUP FOR PUMPING A FLUID AND RECIPROCATING AND ROTATING PUMP DEVICE

(30) Priorité: 22.07.2013 FR 1357189
(43) Date de publication de la demande: 01.06.2016
(73) Titulaire: Eveon, 38330 Montbonnot Saint Martin (FR)
(72) Inventeur: DEHAN, Christophe, F-38330 Saint Ismier (FR); WATTELLIER, Arnaud, F-38320 Eybens (FR)
(74) Mandataire: Prugneau, Philippe
(86) Numéro de dépôt international: PCT/FR2014/051413
(87) Numéro de publication internationale: WO 2015/011352

(56) Documents cités:
- WO-A1-2007/045516
- DE-A1- 3 441 215
- DE-A1-102011 076 751
- US-A- 4 008 003
- US-A- 5 494 420
- US-B1- 6 318 974

## Description

### Domaine technique

L'invention concerne de façon générale un sous-ensemble oscillo-rotatif pour pompage d'un fluide et un dispositif de pompage oscillo-rotatif pour pompage volumétrique d'un fluide.

### Technique antérieure

L'utilisation de dispositifs de pompage volumétrique pour la production et/ou la reconstitution (mélanges liquide-solide ou liquide-liquide) et/ou l'administration (injection, infusion, oral, spray, ...), est connue, notamment pour des applications médicales, esthétiques, vétérinaires. Pour ce type d'applications, il convient de pomper de manière contrôlée des quantités précises de fluide, mais il est aussi souvent nécessaire de raccorder l'arrivée et la sortie en fluide du dispositif de pompage à des dispositifs fluidiques additionnels, par exemple un distributeur fluidique permettant de transférer le fluide vers un ou plusieurs contenants ou dispositifs d'administration afin de réaliser plusieurs fonctions fluidiques avec une unique pompe.

Les dispositifs de pompage connus présentent généralement des conduits disposés à 180° l'un de l'autre. Aussi, le raccordement fluidique de ces dispositifs de pompage avec d'autres dispositifs fluidiques se fait par l'intermédiaire de tuyaux, souples ou rigides, qui sont encombrants, risquent d'être accrochés et arrachés lors des manipulations. Les dispositifs de pompage sont ainsi volumineux et peu pratiques à l'emploi ce qui est préjudiciable à leur utilisation, en particulier pour les applications dans le domaine médical. Un tel dispositif ce pompage est connu par les publications DE 34 41 215 A1 ou US 3,168,872 qui décrivent chacun un dispositif de pompage oscillo-rotatif comportant un corps creux définissant une cavité et dont la paroi est traversée par deux conduits débouchant dans la cavité et disposés à 180° l'un de l'autre. Ce dispositif de pompage oscillo-rotatif comporte également un piston logé dans la cavité dans laquelle il est mobile angulairement et en translation axiale alternative pour faire varier les dimensions de la chambre de travail qu'il définit avec la cavité. Le piston comporte un méplat apte à être successivement en communication avec l'un, puis aucun, puis l'autre des conduits. Ainsi, le fluide peut être aspiré par l'un des conduits, stocké dans la chambre de travail, puis refoulé par l'autre conduit.

### Exposé de l'invention

Le but de l'invention est de remédier à ces inconvénients en proposant un sous-ensemble oscillo-rotatif pour pompage et un dispositif de pompage oscillo-rotatif autorisant une orientation angulaire relative des conduits d'admission et de refoulement différente de 180°, d'un coût de fabrication modéré avec un nombre de pièces limité, réversible, précis, permettant le transfert de liquide visqueux même à haute pression et ayant un bon rendement fluidique et énergétique.

A cet effet, l'invention a pour objet un sous-ensemble oscillo-rotatif pour pompage d'un fluide notamment à usage médical comportant un corps creux définissant une cavité d'axe longitudinal et dont la paroi est traversée par deux conduits débouchant radialement dans ladite cavité, un piston logé dans ladite cavité avec laquelle il définit une chambre de travail, le piston étant mobile angulairement entre des configurations fluidiques dans lesquelles il autorise la communication fluidique entre la chambre de travail et l'un des conduits et des configurations de commutation dans lesquelles il interdit toute communication fluidique avec chacun des conduits, le piston étant mobile alternativement en translation longitudinale de sorte à faire varier les dimensions de la chambre de travail et successivement aspirer puis refouler le fluide par l'un puis l'autre des conduits, caractérisé en ce que les conduits sont décalés l'un de l'autre longitudinalement, en ce que le piston comporte un canal débouchant longitudinalement dans la chambre de travail et radialement dans au moins une paire d'évidements latéraux prévus sur la périphérie du piston décalés longitudinalement l'un de l'autre et agencés de sorte que, dans chaque configuration fluidique, seul l'un des évidements est en communication fluidique avec l'un seulement des conduits et en ce que les évidements de la paire sont décalées angulairement entre eux d'un angle différent de 180° et en ce que les conduits sont décalées angulairement entre eux du même angle.

L'idée à la base de l'invention est de prévoir les évidements du piston à des hauteurs différentes de sorte qu'ils puissent être orientés angulairement selon toute configuration optimale pour le raccordement fluidique des conduits du corps.

Le sous-ensemble oscillo-rotatif selon l'invention peut avantageusement présenter les particularités suivantes :
- les évidements sont superposées entre eux dans un plan longitudinal et en ce que les conduits sont superposées entre eux dans un plan longitudinal ;
- le piston comporte n paires d'évidements répartis dans deux plans radiaux disjoints, et le canal est agencé pour déboucher radialement dans les n évidements de sorte que lors d'une révolution complète du piston dans le corps, le piston soit n fois dans chacune des configurations fluidiques ;
- il comporte au moins un premier et un second étages à chacun desquels correspond de manière distincte un ensemble de deux conduits, une chambre de travail, un canal et une paire d'évidements ;
- les chambres de travail présentent des sections différentes ;
- il comporte au moins une came et un doigt de guidage, portés l'un par le piston, l'autre par le corps, et agencés pour coopérer réciproquement de sorte que la rotation du piston par rapport au corps provoque :
   sur une première portion angulaire, la translation axiale dans un premier sens du piston par rapport au corps,
   sur une seconde portion angulaire, l'immobilisation axiale du piston par rapport au corps,
   sur une troisième portion angulaire, la translation axiale dans un second sens du piston par rapport au corps,
   sur une quatrième portion angulaire, l'immobilisation axiale du piston par rapport au corps,
   les conduits et les évidements étant agencés pour que les conduits soient obturés pendant les seconde et quatrième portions angulaires

L'invention s'étend à un dispositif de pompage oscillo-rotatif pour fluide notamment à usage médical, caractérisé en ce qu'il comporte des moyens d'entrainement et un sous-ensemble oscillo-rotatif pour pompage d'un fluide tel que décrit et des moyens de couplage mécanique amovibles pour raccorder mécaniquement lesdits moyens d'entrainement audit piston de manière démontable.

Ce dispositif de pompage oscillo-rotatif peut comporter un sous-ensemble oscillo-rotatif pour pompage d'un fluide comportant au moins un premier et un second étages à chacun desquels correspond de manière distincte un ensemble de deux conduits, une chambre de travail, un canal et une paire d'évidements, les chambres de travail présentant des sections différentes, l'un des conduits est raccordé à l'arrivée d'un premier fluide, l'un des conduits est raccordé à l'arrivée d'un second fluide, en ce que le dispositif de pompage oscillo-rotatif comporte des moyens de mélange du premier fluide refoulé par l'autre des conduits et du second fluide refoulé par l'autre des conduits, le mélange obtenu étant proportionnel à la différence de sections entre les chambres de travail.

### Présentation sommaire des dessins

La présente invention sera mieux comprise et d'autres avantages apparaîtront à la lecture de la description détaillée de deux modes de réalisation pris à titre d'exemples nullement limitatifs et illustrés par les dessins annexés, dans lesquels :
- la figure 1 est une vue de coté du piston du sous-ensemble oscillo-rotatif pour pompage selon un premier mode de réalisation de l'invention ;
- les figures 2 à 7 sont des vues de coté en transparence du sous-ensemble oscillo-rotatif selon le premier mode de réalisation de l'invention, illustré en six positions de fonctionnement distinctes lors d'un cycle de pompage (admission, commutation, refoulement, commutation) ;
- la figure 8 est une vue en coupe du corps et du piston du sous-ensemble oscillo-rotatif pour pompage selon un second mode de réalisation de l'invention ;
- les figures 9 à 14 sont des vues en coupe du sous-ensemble oscillo-rotatif selon le second mode de réalisation de l'invention, le piston n'étant pas illustré en coupe, le sous-ensemble oscillo-rotatif étant illustré en six positions de fonctionnement distinctes lors d'un cycle de pompage.

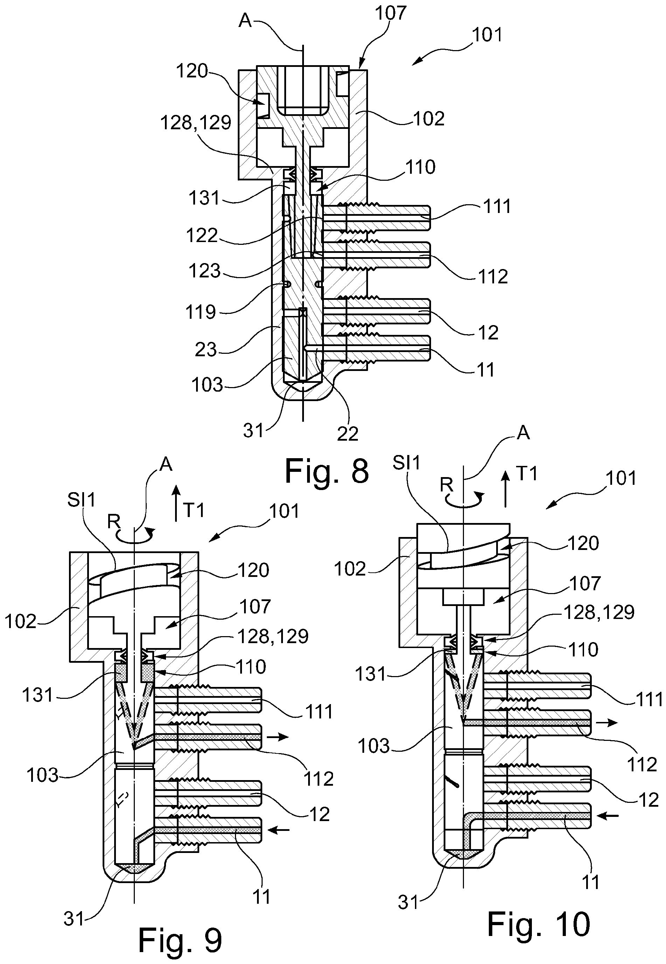

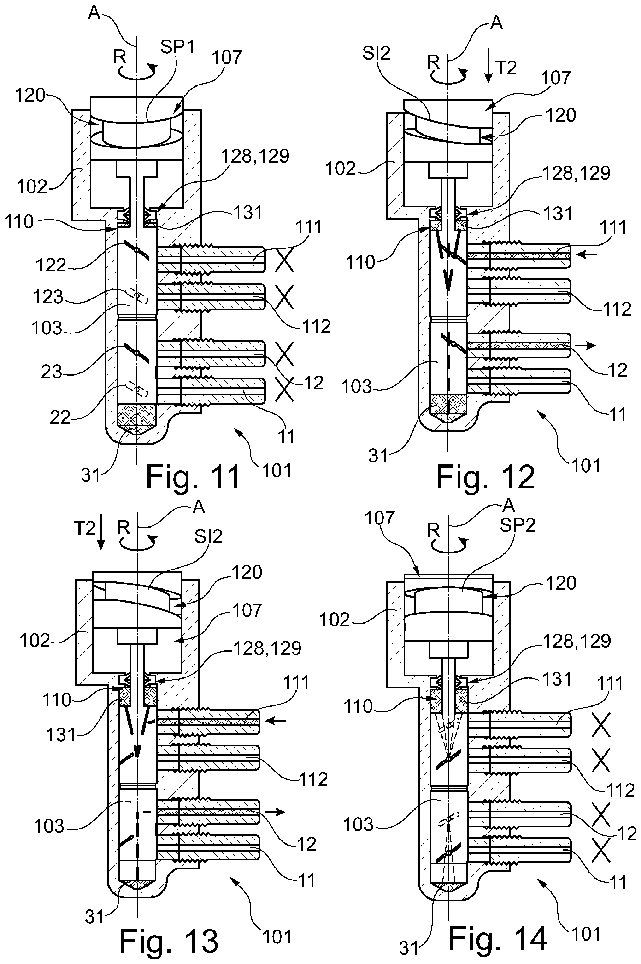

Sur les figures 8 à 14, les éléments analogues à ceux des figures précédentes y sont affectés des mêmes numéros de référence, augmentés de 100.

### Description des modes de réalisation

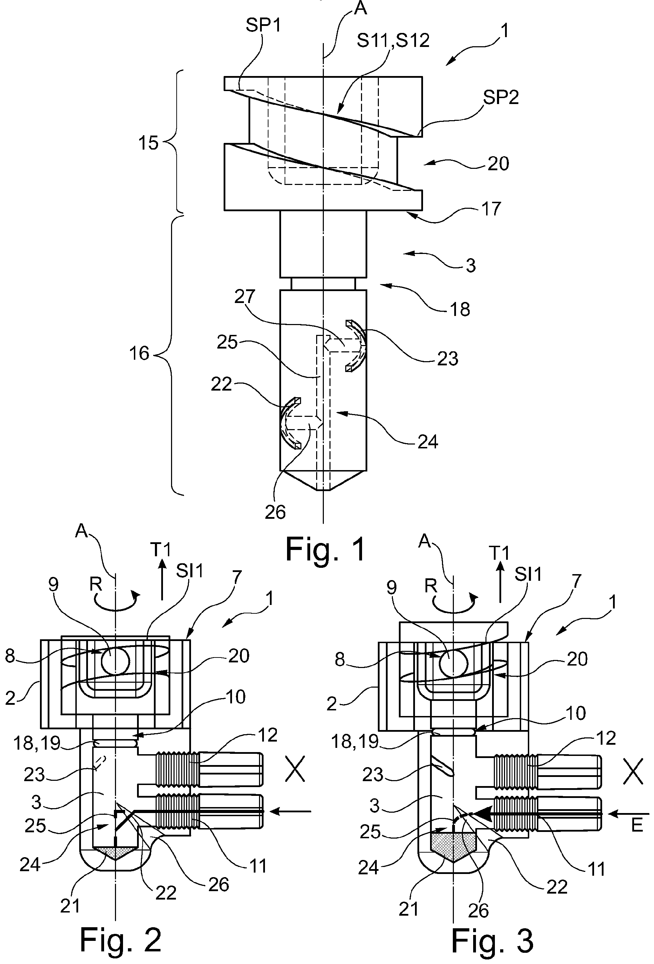

Le sous-ensemble oscillo-rotatif pour pompage selon l'invention peut présenter une configuration simple-effet à étage unique, décrite ci-après en tant que premier mode de réalisation illustré par les figures 1 à 7, et une configuration multi-effet à plusieurs étages, par exemple la configuration double-effet décrite plus loin en tant que second mode de réalisation illustré par les figures 8 à 14.

En référence aux figures 2 à 7, le sous-ensemble oscillo-rotatif 1 selon le premier mode de réalisation de l'invention comprend un corps 2, un piston 3 et un doigt de guidage 9.

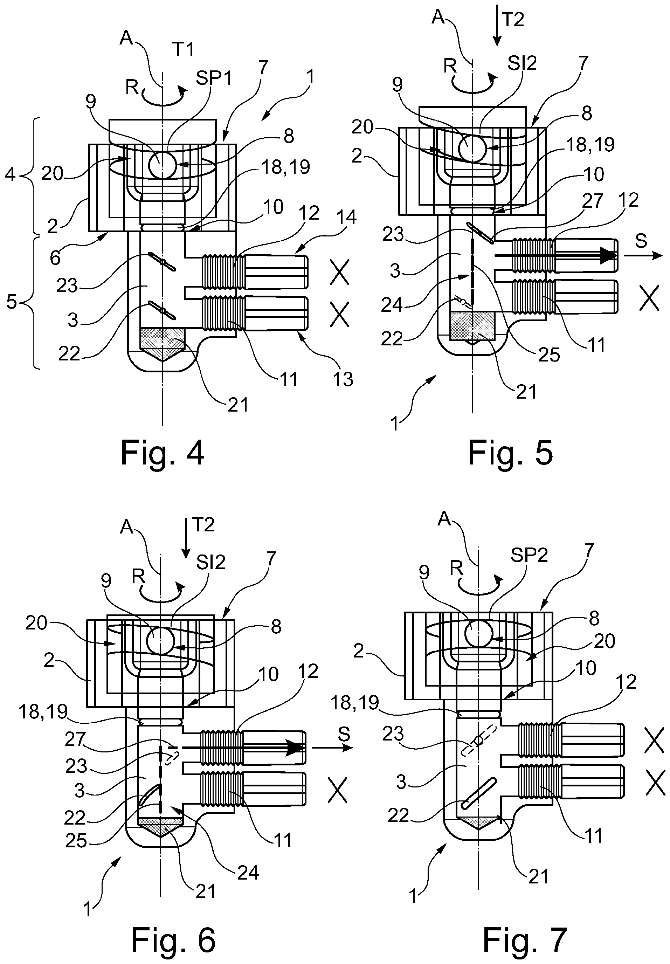

Le corps 2 est creux et formé de deux portions cylindriques 4, 5 (visibles sur la figure 4), de diamètres différents, reliées entre elles par un épaulement 6. Le corps 2 est par exemple réalisé en matériau plastique ou en tout autre matériau adapté.

L'intérieur de la portion cylindrique 4 de grand diamètre forme un alésage 7 d'axe longitudinal A. L'extrémité libre de cette portion cylindrique 4 de grand diamètre est ouverte et destinée à recevoir l'engagement longitudinal du piston 3. L'autre extrémité est raccordée à la portion cylindrique de petit diamètre par l'épaulement 6. La paroi de la portion cylindrique 4 de grand diamètre est traversée par un orifice 8 destiné à recevoir un doigt de guidage 9 radial disposé de sorte à dépasser dans l'alésage 7. Le doigt de guidage 9 est par exemple une goupille solidarisée au corps par déformation élastique et/ou par collage et/ou par tout autre moyen adapté. Le doigt de guidage 9 présente par exemple une section cylindrique ou toute autre section adaptée.

L'intérieur de la portion cylindrique 5 de petit diamètre définit une cavité 10 d'axe longitudinal A et de diamètre inférieur à celui de l'alésage 7. L'extrémité libre de la portion cylindrique 5 de petit diamètre est fermée et forme le fond du corps 2. L'alésage 7 et la cavité 10 sont destinés à recevoir le piston 3 logé dans le corps 2. L'extrémité libre du piston 3 définit ainsi, avec le corps 2, une chambre de travail 21. La paroi de la portion cylindrique 5 de petit diamètre est traversée par deux conduits 11, 12 décalés longitudinalement l'un de l'autre et débouchant radialement dans la cavité 10. Dans l'exemple illustré, les conduits 11, 12 sont longitudinalement superposés l'un à l'autre et donc décalés angulairement d'un angle de 0°. Selon un autre mode de réalisation non représenté, ces conduits peuvent être décalés angulairement l'un par rapport à l'autre de tout autre angle approprié, par exemple de 90°. Les conduits 11, 12 ont par exemple une section circulaire et présentent un même diamètre. Le corps 2 comporte des embouts de raccordement 13, 14 (visibles sur la figure 4) entourant chacun un conduit 11, 12 et aptes à être raccordés par exemple à un distributeur fluidique par l'intermédiaire d'un tuyau d'admission ou de refoulement (non représenté).Les embouts de raccordement 13, 14 peuvent également être raccordés au distributeur fluidique ou intégrés directement dans le distributeur fluidique. Pour les besoins de la description, on utilise ci-après de manière non limitative les termes conduit 11 d'admission et conduit 12 de refoulement pour distinguer les conduits 11, 12 entre eux. Bien entendu, selon la configuration de fonctionnement choisie, chacun des conduits 11, 12 peut indifféremment servir pour l'admission ou pour le refoulement du fluide.

En référence en particulier à la figure 1, le piston 3 est formé de deux portions cylindriques 15, 16 de diamètres différents reliées entre elles par un épaulement 17. Les portions cylindriques 16, 15 de petit et grand diamètres du piston 3 présentent respectivement un diamètre extérieur légèrement inférieur aux diamètres de la cavité 10 et de l'alésage 7 dans lesquels le piston 3 peut ainsi être logé. Le piston 3 est par exemple réalisé en matériau plastique ou en tout autre matériau adapté.

Le piston 3 comporte une rainure périphérique 18 prévu sur la portion cylindrique 16 de petit diamètre, destinée à recevoir un joint d'étanchéité 19 en contact avec la paroi intérieur de la cavité 10. Ce joint d'étanchéité 19 est réalisé dans un matériau présentant un module d'élasticité inférieur à ceux du piston 3 et du corps 2. Il est par exemple réalisé en élastomère, en silicone, en caoutchouc, en élastomère TPE ou en tout autre matériau adapté.

Le piston 3 comporte également une gorge 20 définissant une double came de guidage du doigt de guidage 9. La hauteur de cette gorge 20, dans le sens longitudinal, est ajustée aux dimensions du doigt de guidage 9 pour autoriser son guidage sans jeu excessif. La gorge 20 comporte une première et une seconde portion inclinée SI1, SI2, symétriques l'une de l'autre par rapport à un plan longitudinal médian. Les première et seconde portions inclinées SI1, SI2 présentent ainsi des pentes inversée sur la périphérie du piston 3. La première et la seconde portion inclinée SI1, SI2 sont séparées l'une de l'autre par des première et seconde portions planes SP1, SP2 sensiblement parallèles entre elles et perpendiculaires à l'axe longitudinal A. Ainsi, par l'intermédiaire du doigt de guidage 9 et de la gorge 20, la rotation dans un premier sens de rotation R du piston 3 par rapport au corps 2, provoque successivement la translation axiale du piston 3 par rapport au corps 2 dans un premier sens de translation T1 le long de la première portion inclinée SI1, puis l'immobilité axiale du piston 3 par rapport au corps 2 le long de la première portion plane SP1, puis la translation axiale du piston 3 par rapport au corps 2 dans un second sens de translation T2 le long de la seconde portion inclinée SI2, puis enfin l'immobilité axiale du piston 3 par rapport au corps 2 le long de la seconde portion plane SP2, et ainsi de suite. Le piston 3 oscille ainsi entre une position haute (Cf. figure 4) dans laquelle la chambre de travail 21 présente un volume maximal et une position basse (Cf. figure 7) dans laquelle la chambre de travail 21 présente un volume minimal. Entre ces deux positions du piston 3, la chambre de travail 21 admet puis refoule le fluide.

Le piston comporte deux évidements 22, 23 latéraux prévus sur la périphérie de la portion cylindrique 16 de petit diamètre du piston 3. Ces évidements 22, 23 sont décalés angulairement l'un de l'autre d'un second angle ici de 180°, et décalés longitudinalement d'une distance dépendant notamment du profil de la gorge 20 et prévue de sorte que, à tout moment, au maximum un seulement des évidements 22, 23 est en regard de l'un seulement des conduits 11, 12. Lorsque les conduits 11, 12 sont décalés l'un de l'autre d'un angle différent de 0°, les évidements 22, 23 sont décalés angulairement et longitudinalement en conséquence, en tenant compte également du profil de la gorge 20. Pour les besoins de la description, on utilise ci-après de manière non limitative les thermes évidement 22 d'admission et évidement 23 de refoulement pour distinguer les évidements 22, 23 entre eux. Bien entendu, selon la configuration de fonctionnement choisie, chacun des évidements peut indifféremment servir pour l'admission ou pour le refoulement du fluide.

Le piston comporte un canal 24, également prévu dans la portion cylindrique 16 de petit diamètre. En référence à la figure 1, le canal 24, comporte un tronçon longitudinal 25 débouchant longitudinalement dans la chambre de travail 31 et deux tronçons radiaux 26, 27 débouchant chacun radialement dans l'un des évidements 22, 23.

Dans l'exemple illustré, les évidements 22, 23 présentent chacun une forme de rainure inclinée. Le sens d'inclinaison est inversé d'une rainure 22, 23 à l'autre sur le développé du piston 3. L'inclinaison des rainures 22, 23 est directement liée au sens de la pente de la gorge 20 de sorte que, pendant chacune des phases d'admission et de refoulement, le canal 24 débouche par l'un des évidements 22, 23, en communication fluidique, avec respectivement le conduit 11 d'admission ou avec le conduit 12 de refoulement respectif. Ainsi, les rainures 22, 23 inclinées correspondent au profil minimal des évidements permettant de limiter le volume mort du sous-ensemble oscillo-rotatif 1. Selon d'autres modes de réalisation non représentés, les évidements 22, 23 peuvent par exemple présenter un méplat ou tout autre profil adapté.

L'extrémité libre de la portion cylindrique 15 de grand diamètre du piston 3 présente par exemple une forme en creux (non représentée) destinée à raccorder mécaniquement le piston 3 à des moyens d'entrainement en rotation (non représentés).

Le sous-ensemble oscillo-rotatif 1 simple effet est ainsi pourvu d'un étage unique comportant deux conduits 11, 12, une chambre de travail 31, un canal 24 avec un tronçon longitudinal 25 et deux tronçons radiaux 26, 27, deux évidements 22, 23. Ainsi, à une paire de conduits 11, 12 dits d'admission et de refoulement, correspond une paire d'évidements 22, 23

Selon un mode de réalisation non représenté, le piston comporte n paires d'évidements, chaque paire comportant un évidement d'admission situé dans le même plan radial que l'évidement d'admission précédent et un évidement de refoulement situé dans le même plan radial que l'évidement de refoulement précédent. Les n évidements d'admission sont angulairement disposés entre eux de manière similaire aux n évidements de refoulement entre eux. Les n évidements d'admission sont bien entendu décalés angulairement par rapport aux n évidements de refoulement de sorte que chaque configuration d'admission ne corresponde pas à une configuration de refoulement et inversement. Ainsi, lors d'une révolution complète du piston dans le corps, le piston est n fois dans une configuration d'admission et n fois dans une configuration de refoulement. Entre chaque configuration d'admission et de refoulement, le piston est dans une position intermédiaire correspondant à une configuration de commutation. Pour assurer un tel fonctionnement, la came présente n première et seconde portions inclinées séparées entres elles de portions planes.

Pour faire fonctionner le sous-ensemble oscillo-rotatif 1 simple effet, le conduit 11 d'admission est raccordé à un tuyau d'alimentation en fluide (non représenté), le conduit de refoulement est raccordé à un tuyau de refoulement fluide (non représenté) de ce même fluide, et le piston 3 est mécaniquement raccordé à des moyens d'entrainement en rotation (non représentés) de type connu. Le fait que les conduits 11, 12 d'admission et de refoulement soient superposés longitudinalement l'un à l'autre permet de faciliter le raccordement du sous-ensemble oscillo-rotatif 1 à tout autre dispositif fluidique. En effet, un distributeur fluidique peut par exemple être prévu dans l'alignement direct des conduits 11, 12 d'admission et de refoulement, sans nécessiter de tuyau contournant le sous-ensemble oscillo-rotatif 1, voire même directement sans tuyau.

Le fonctionnement du sous-ensemble oscillo-rotatif 1 simple-effet selon l'invention est décrit ci-après en référence aux figures 6 à 11.

En phase admission illustrée par les figures 2 et 3,le doigt de guidage 9 circule le long de la première portion inclinée SI1 de la gorge 20 qui transforme la rotation R du piston 3 en une première translation T1 selon un premier sens de déplacement du piston 3 par rapport au corps 2 qui fait passer le piston 3 d'une position basse (figure 7) dans laquelle la chambre de travail 31 présente un volume minimal, à une position haute (figure 4) dans laquelle la chambre de travail 31 présente un volume maximal. Pendant cette première translation T1, le piston 3 tourne par rapport au corps 2 avec la l'évidement 22 d'admission circulant devant l'orifice du conduit 11 d'admission. Ainsi le conduit 11 d'admission est en communication fluidique avec la chambre de travail 31 par l'intermédiaire de l'évidement 22 d'admission et des tronçons radiaux 26 et longitudinaux 25 du canal 24. Le fluide est aspiré, par dépression, par l'augmentation du volume de la chambre de travail 31 selon la flèche E. Pendant cette phase d'admission, l'évidement 23 de refoulement circule devant la paroi de la cavité 10, sans être en regard du conduit 12 de refoulement. Ainsi, pendant la phase d'admission, le fluide ne sort pas de la chambre de travail 31 par le conduit 12 de refoulement, ce qui est schématisé par une croix. La rotation R du piston 3 par rapport au corps 2 est prolongée jusqu'à atteindre une première phase de commutation.

Dans cette première phase de commutation illustrée par la figure 4, le doigt de guidage 9 circule le long de la première portion plane SP1 de la gorge 20. La rotation R du piston 3 ne provoque alors pas sa translation et le piston 3 est axialement immobile dans sa position haute. Ainsi, le volume de la chambre de travail 31 ne varie pas et reste maximal. Lors de cette phase de commutation, l'évidement 22 d'admission et l'évidement 23 de refoulement sont chacun en regard de la cavité 10, interdisant toute communication fluidique entre la chambre de travail 31 et l'un ou l'autre des conduits 11, 12 d'admission ou de refoulement. Ainsi la chambre de travail 31 est fluidiquement fermée de manière étanche. La rotation R du piston 3 par rapport au corps 2 est prolongée jusqu'à atteindre la phase de refoulement.

Dans cette phase de refoulement illustrée par les figures 5 et 6, le doigt de guidage 9 circule le long de la seconde portion inclinée SI2 de la gorge 20 qui transforme la rotation R du piston 3 en une second translation T2 selon un second sens de déplacement opposé au premier sens de déplacement lors de translation T1. Ainsi, le piston 3 passe de sa position haute (figure 4) à sa position basse (figure 7). Pendant cette seconde translation T2, le piston 3 tourne par rapport au corps 2 avec l'évidement 23 de refoulement circulant devant l'orifice du conduit 12 de refoulement. Ainsi le conduit 12 de refoulement est en communication fluidique avec la chambre de travail 31 par l'intermédiaire et de l'évidement 23 et des tronçons radiaux 27 et longitudinaux 25 du canal 24. Le fluide est refoulé selon la flèche S par surpression par réduction du volume de la chambre de travail 31 par le conduit 12 de refoulement. Pendant cette phase de refoulement, l'évidement 22 d'admission circule devant la paroi de la cavité 10, sans être en regard du conduit 11 d'admission. Ainsi, pendant la phase de refoulement, le fluide n'entre pas dans la chambre de travail 31 par le conduit 11 d'admission. La rotation R du piston 3 par rapport au corps 2 est prolongée jusqu'à atteindre une seconde phase de commutation.

Cette seconde phase de commutation illustrée par la figure 7 est sensiblement similaire à la première phase de commutation. Elle s'en différencie par le piston 3 en position basse, la chambre de travail 31 qui présente un volume minimal et la position des évidements 22, 23 d'admission et de refoulement par rapport aux conduits 11, 12 d'admission et de refoulement, diamétralement inversée par rapport à la première phase de commutation.

Le cycle oscillo-rotatif peut être répété. Il est bien entendu que, selon le sens de rotation du piston 3 par rapport au corps 2, le conduit dit d'admission peut correspondre au conduit de refoulement et inversement.

En modifiant les profils des première et seconde portions inclinées SI1, SI2 ainsi que celui des évidements 22, 23 d'admission et de refoulement, le ratio entre la phase d'admission et la phase de refoulement peut être ajusté. On peut ainsi allonger la durée de l'une ou l'autre de ces phases d'admission et de refoulement par rapport à l'autre.

Le sous-ensemble oscillo-rotatif 101 selon le second mode de réalisation de l'invention est illustré par les figures 8 à 14 et présente une configuration double-effet. A cet effet, il comporte deux étages, un premier étage similaire à celui du sous-ensemble oscillo-rotatif 1 et un second étage comportant deux conduits 111, 112 d'admission et de refoulement, une chambre de travail 131, un canal 124 disjoint du canal 24 du premier étage, et des évidements 122, 123 d'admission et de refoulement tels que ceux du premier étage. Ainsi, à chaque paire de conduits 11, 12, 111, 112 d'admission et de refoulement, correspond une paire d'évidements 22, 23, 122, 123 d'admission et de refoulement.

Dans l'exemple illustré, les évidements 22, 122 d'admission sont longitudinalement séparés par les évidements 23, 123 de refoulement et les conduits d'admission et de refoulement 11, 12, 111, 112 sont longitudinalement superposés entre eux. De plus, l'évidement 22 d'admission du premier étage est longitudinalement superposé à l'évidement 123 de refoulement du second étage, et l'évidement 23 de refoulement du premier étage est longitudinalement superposé à l'évidement 122 de refoulement du second étage. Le sous-ensemble oscillo-rotatif 101 permet ainsi d'être simultanément en phase d'admission pour un étage et en phase de refoulement pour l'autre étage.

Comme détaillé sur la figure 8, le canal 124 du second étage présente une forme tronconique s'évasant vers la chambre de travail 131.

Le corps 102 comporte une cavité 110 présentant longitudinalement une hauteur supérieure permettant de loger les deux étages. Dans ce mode de réalisation, le joint d'étanchéité 119 est prévu entre les deux étages. De plus, le corps 103 comporte un sillon annulaire 128 prévu entre l'alésage 107 et la cavité 110, recevant un joint d'étanchéité complémentaire 129.

Selon une première configuration, les conduits 11, 111 dits d'admission des premier et second étages peuvent être reliés fluidiquement à une arrivée commune d'un même fluide et les conduits 12, 112 dits de refoulement des premier et second étages peuvent être reliés flluidiquement à une sortie commune du même fluide.

Selon une seconde configuration le conduit 12, 112 dit de refoulement d'un étage peut être raccordé fluidiquement au conduit 11, 111 dit d'admission de l'autre étage.

Selon une troisième configuration, le sous-ensemble oscillo-rotatif double-effet peut avantageusement être utilisé pour réaliser des dosages proportionnels par tour en utilisant un étage pour un premier fluide et un autre étage pour un second fluide. Pour obtenir optionnellement un mélange proportionnel, les conduits 12, 112 dits de refoulement de chaque étage étant par exemple raccordés à un même contenant destiné à recevoir les deux fluides. En modifiant le ratio entre les sections des chambres de travail 31, 131, on peut faire varier proportionnellement le dosage entre les deux fluides, la course étant toujours identique pour les deux chambres de travail 31, 131.

Dans ces trois configurations, le débit du dispositif de pompage intégrant un tel sous-ensemble oscillo-rotatif 101 double-effet sera augmenté, avec une fréquence de pulsation doublée, par rapport à un sous-ensemble oscillo-rotatif 1 simple-effet.

Selon une quatrième configuration, les deux étages peuvent être identiques et simplement décalés l'un de l'autre longitudinalement. Ainsi, les deux phases d'admission des deux étages sont concomitantes entre elles, et les deux phases de refoulement des deux étages sont concomitantes entre elles. Dans ce cas, le débit du dispositif de pompage intégrant un tel sous-ensemble oscillo-rotatif 101 double-effet sera doublé avec une fréquence de pulsation identique par rapport à un sous-ensemble oscillo-rotatif 1 simple-effet.

L'invention permet d'atteindre les objectifs précédemment mentionnés. En effet, le sous-ensemble oscillo-rotatif 1, 101 selon l'invention permet l'obtention des conduits d'admission et de refoulement avec une orientation angulaire relative différente de 180°, par exemple de 0°, ou de tout autre angle adapté. Les conduits d'admission et de refoulement peuvent également, si nécessaire, être orientés à 180°. Ainsi, le sous-ensemble oscillo-rotatif 1, 101 peut facilement être raccordé à tout autre dispositif fluidique avec un circuit fluidique simple et direct.

Par ailleurs, le sous-ensemble oscillo-rotatif 1, 101 selon l'invention est réversible, par simple inversion du sens de rotation du piston 3, 103. Ainsi le conduit 11, 111 dit d'admission devient conduit 12, 112 dit de refoulement et inversement. Le découplage mécanique entre le piston 3, 103 et les moyens d'entrainement permettent d'obtenir un sous-ensemble oscillo-rotatif jetable alors que la partie motrice est réutilisable. On assure ainsi, à moindre coût, la stérilité du sous-ensemble oscillo-rotatif 1, 101 en le remplaçant entre deux utilisations. Ainsi, seule la partie fluidique du dispositif de pompage oscillo-rotatif est à renouvelée, les parties motorisation et commande étant conservées entre deux usages. Le fait que les efforts axiaux soient transmis par la came, permet d'utiliser des moyens d'entrainement limités à la rotation, et des moyens de couplage mécanique entre le piston 3 et ces moyens d'entrainement limités à la simple transmission d'un couple. La gorge 20, 120et le doigt de guidage 9 permet par ailleurs de s'assurer que la translation alternative du piston 3 est synchrone avec la rotation du même piston 3.

Le sous-ensemble oscillo-rotatif 1, 101 selon l'invention interdit toute circulation fluidique avec les conduits 11, 111, 12, 112 dits d'admission et de refoulement pendant les phases de commutation, sans pour autant créer d'effet de surpression ou de dépression par blocage hydraulique pendant ces phases. Les évidements 22, 23, 122, 123 inclinés permettent de plus de limiter le volume mort.

Le contact entre le joint d'étanchéité et le corps permet de caler angulairement le sous-ensemble oscillo-rotatif 1, 101 en usine lors de son montage initial. Ce calage angulaire sera ainsi facilement conservé jusqu'à la mise en service du sous-ensemble oscillo-rotatif 1, 101 dans le dispositif oscillo-rotatif. Il est néanmoins possible de prévoir un repère visuel de la position angulaire du piston 3, 103 par rapport au corps 2, 102 ou un capteur de toute technologie adaptée.

Il va de soi que la présente invention ne saurait être limitée à la description qui précède d'un de ses modes de réalisation, susceptibles de subir quelques modifications sans pour autant sortir du cadre de l'invention, tel que défini par l'objet respectif des revendications 1 à 8.

## Revendications

1. Sous-ensemble oscillo-rotatif (1 ; 101) pour pompage d'un fluide notamment à usage médical comportant un corps (2 ; 102) creux définissant une cavité (10 ; 110) d'axe longitudinal (A) et dont la paroi est traversée par deux conduits (11, 12 ; 111, 112) débouchant radialement dans ladite cavité (10 ; 110), un piston (3 ; 103) logé dans ladite cavité (10 ; 110) avec laquelle il définit une chambre de travail (31 ; 131), ledit piston (3 ; 103) étant mobile angulairement entre des configurations fluidiques dans lesquelles il autorise la communication fluidique entre ladite chambre de travail (31 ; 131) et l'un desdits conduits (11, 12 ; 111, 112) et des configurations de commutation dans lesquelles il interdit toute communication fluidique avec chacun desdits conduits, ledit piston (3 ; 103) étant mobile alternativement en translation longitudinale de sorte à faire varier les dimensions de ladite chambre de travail (31 ; 131) et successivement aspirer puis refouler ledit fluide par l'un puis l'autre desdits conduits (11, 12 ; 111, 112), **caractérisé en ce que** lesdits conduits (11, 12 ; 111, 112) sont décalés l'un de l'autre longitudinalement, **en ce que** ledit piston (3 ; 103) comporte un canal (25 ; 125) débouchant longitudinalement dans ladite chambre de travail (31 ; 131) et radialement dans au moins une paire d'évidements (22, 23 ; 122, 123) latéraux prévus sur la périphérie dudit piston (3 ; 103) décalés longitudinalement l'un de l'autre et agencés de sorte que, dans chaque configuration fluidique, seul l'un desdits évidements (22, 23 ; 122, 123) est en communication fluidique avec l'un seulement desdits conduits (11, 12 ; 111, 112) et **en ce que** lesdits évidements (22, 23 ; 122, 123) de ladite paire sont décalées angulairement entre eux d'un angle différent de 180° et **en ce que** lesdits conduits (11, 12 ; 111, 112) sont décalées angulairement entre eux du même angle.

2. Sous-ensemble oscillo-rotatif (1 ; 101) pour pompage selon la revendication 1, **caractérisé en ce que** lesdits évidements (22, 23 ; 122, 123) sont superposées entre eux dans un plan longitudinal et **en ce que** lesdits conduits (11, 12 ; 111, 112) sont superposées entre eux dans un plan longitudinal.

3. Sous-ensemble oscillo-rotatif pour pompage selon la revendication 1, **caractérisé en ce que** ledit piston comporte n paires d'évidements répartis dans deux plans radiaux disjoints, **en ce que** ledit canal est agencé pour déboucher radialement dans lesdits n évidements de sorte que lors d'une révolution complète dudit piston dans ledit corps, ledit piston soit n fois dans chacune desdites configurations fluidiques.

4. Sous-ensemble oscillo-rotatif (101) pour pompage selon la revendication 1, **caractérisé en ce qu'**il comporte au moins un premier et un second étages à chacun desquels correspond de manière distincte un ensemble de deux conduits (11, 12, 111, 112), une chambre de travail (31 ; 131), un canal (25, 125) et une paire d'évidements (22, 23 ; 122, 123).

5. Sous-ensemble oscillo-rotatif (101) pour pompage selon la revendication 5, **caractérisé en ce que** lesdites chambres de travail (31 ; 131) présentent des sections différentes.

6. Sous-ensemble oscillo-rotatif (1 ; 101) pour pompage selon la revendication 1, **caractérisé en ce qu'**il comporte au moins une came et un doigt de guidage (9), portés l'un par ledit piston (3 ; 103), l'autre par ledit corps (2 ; 102), et agencés pour coopérer réciproquement de sorte que la rotation dudit piston (3 ; 103) par rapport audit corps (2 ; 102) provoque :
- sur une première portion angulaire, la translation axiale (T1) dans un premier sens dudit piston (3 ; 103) par rapport audit corps (2 ; 102),
- sur une seconde portion angulaire, l'immobilisation axiale dudit piston (3 ; 103) par rapport audit corps (2 ; 102),
- sur une troisième portion angulaire, la translation axiale dans un second sens dudit piston (3 ; 103) par rapport audit corps (2 ; 102),
- sur une quatrième portion angulaire, l'immobilisation axiale dudit piston (3 ; 103) par rapport audit corps (2 ; 102),
lesdits conduits (11, 12 ; 111, 112) et lesdits évidements (22, 23 ; 122, 123) étant agencés pour que lesdits conduits (11, 12 ; 111, 112) soient obturés pendant lesdites seconde et quatrième portions angulaires.

7. Dispositif de pompage oscillo-rotatif pour fluide notamment à usage médical, **caractérisé en ce qu'**il comporte des moyens d'entrainement et un sous-ensemble oscillo-rotatif (1 ; 101) pour pompage d'un fluide selon l'une quelconque des revendications précédentes et des moyens de couplage mécanique amovibles pour raccorder mécaniquement lesdits moyens d'entrainement audit piston (3 ; 103) de manière démontable.

8. Dispositif de pompage oscillo-rotatif selon la revendication 7, **caractérisé en ce qu'**il comporte un sous-ensemble oscillo-rotatif (101) pour pompage d'un fluide selon la revendications 5, **en ce que** l'un desdits conduits (11, 12) est raccordé à l'arrivée d'un premier fluide, l'un desdits conduits (111, 112) est raccordé à l'arrivée d'un second fluide, **en ce que** ledit dispositif de pompage oscillo-rotatif comporte des moyens de mélange dudit premier fluide refoulé par l'autre desdits conduits (12, 11) et dudit second fluide refoulé par l'autre desdits conduits (112, 111), le mélange obtenu étant proportionnel à la différence de sections entre lesdites chambres de travail (31 ; 131).

## Patentansprüche

1. Oszillierende und rotierende Untereinheit (1; 101) zum Pumpen eines Fluids, insbesondere zur medizinischen Verwendung, umfassend einen hohlen Körper (2; 102), der einen Hohlraum (10; 110) mit einer Längsachse (A) definiert und dessen Wand von zwei Leitungen (11, 12; 111, 112) durchquert wird, die radial in den Hohlraum (10; 110) münden, einen Kolben (3; 103), der In dem Hohlraum (10; 110) aufgenommen Ist, mit dem dieser eine Arbeitskammer (31; 131) definiert, wobei der Kolben (3; 103) winklig zwischen Strömungsanordnungen, in denen dieser dle Fluidverbindung zwischen der Arbeitskammer (31; 131) und einer der Leitungen (11, 12; 111, 12) zulåsst, und Umschaltanordnungen bewegbar Ist, In denen jede Fluidverbindung mit jeder der Leitungen unterbunden ist, wobei der Kolben (3; 103) alternativ translatorisch in Längsrichtung derart bewegbar Ist, das die Abmessungen der Arbeitskammer (31; 131) variiert werden und aufeinanderfolgend das Fluid von der einen, dann von der anderen der Leitungen (11, 12; 111; 112) angesaugt, dann gefördert wird, **dadurch gekennzeichnet, dass** die Leitungen (11, 12; 111, 112) In Längsrichtung voneinander versetzt sind, dass der Kolben (3; 103) einen Kanal (25; 125) umfasst, der In Längsrichtung in die Arbeitskammer (31; 131) und radial In mindestens ein Paar von lateralen Ausnehmungen (22, 23; 122, 123) mündet, die am Umfang des Kolbens (3; 103), in Längsrichtung voneinander versetzt, bereitgesteilt sind und derart angeordnet sind, dass, in jeder Strömungsanordnung, nur eine der Ausnehmungen (22, 23; 122, 123) mit nur einer der Leitungen (11, 12; 111, 112) In Fluidverbindung ist, und dass die Ausnehmungen (22, 23; 122, 123) des Paars winklig zwischen diesen um einen von 180° verschiedenen Winkel versetzt sind, und dass die Leitungen (11, 12; 111, 112) winklig zwischen diesen um denselben Winkel versetzt sind.

2. Oszillierende und rotierende Untereinheit (1; 101) zum Pumpen nach Anspruch 1, **dadurch gekennzeichnet, dass** die Ausnehmungen (22, 23; 122, 123) In einer Längsebene übereinander angeordnet sind, und dass die Leitungen (11, 12; 111, 112) in einer Längsebene übereinander angeordnet sind.

3. Oszillierende und rotierende Untereinheit zum Pumpen nach Anspruch 1, **dadurch gekennzeichnet, dass** der Kolben n Paare von Ausnehmungen aufweist, die in zwei getrennten radialen Ebenen verteilt sind, dass der Kanal angeordnet ist, um radial in die n Ausnehmungen derart zu münden, dass bei einer vollständigen Umdrehung des Kolbens in dem Körper der Kolben n-mal in jeder der Fluidauslegungen ist.

4. Oszillierende und rotierende Untereinheit zum Pumpen (101) nach Anspruch 1, **dadurch gekennzeichnet, dass** diese mindestens eine erste und eine zweite Stufe umfasst, von denen jede auf verschiedene Weise zu einer Gruppe von zwei Leitungen (11, 12, 111, 112), einer Arbeitskammer (31; 131), eines Kanals (25, 125) und eines Paares von Ausnehmungen (22, 23; 122, 123) gehört.

5. Oszillierende und rotierende Untereinheit zum Pumpen (101) nach Anspruch 5, **dadurch gekennzeichnet, dass** die Arbeltskammern (31; 131) verschiedene Querschnitte aufweisen.

6. Oszillierende und rotierende Untereinheit zum Pumpen (1; 101) nach Anspruch 1, **dadurch gekennzeichnet, dass** diese mindestens einen Nocken und einen Führungsstift (9) umfasst, wobei der eine von dem Kolben (3; 103), der andere von dem Körper (2; 102) getragen wird, und die angeordnet sind, um hin- und hergehend derart zusammenzuwirken, dass die Drehung des Kolbens (3; 103) in Bezug auf den Körper (2; 102) bewirkt:
- an einem ersten Winkelabschnitt, die axiale Translation (T1) in einer ersten Richtung des Kolbens (3; 103) in Bezug auf den Körper (2; 102),
- an einem zweiten Winkelabschnitt, die axiale Immobilisierung des Kolbens (3; 103) in Bezug auf den Körper (2; 102),
- an einem dritten Winkelabschnitt, die axiale Translation in einer zweiten Richtung des Kolbens (3; 103) in Bezug auf den Körper (2; 102),
- an einem vierten Winkelabschnitt, die axiale Immobilisierung des Kolbens (3; 103) in Bezug auf den Körper (2; 102), wobei die Leitungen (11, 12; 111, 112) und die Ausnehmungen (22, 23; 122, 123) angeordnet sind, um die Leitungen (11, 12; 111, 112) während des zweiten und vierten Winkelabschnltts zu verschließen.

7. Oszillierende und rotierende Vorrichtung zum Pumpen für ein Fluid, insbesondere zur medizinischen Verwendung, **dadurch gekennzeichnet, dass** diese Antriebsmittel und eine Oszillierende und rotierende Untereinheit (1; 101) zum Pumpen eines Fluids nach einem der vorhergehenden Ansprüche und lösbare mechanische Kupplungsmittel, um die Antriebsmittel mit dem Kolben (3; 103) auf abnehmbare Weise mechanisch zu verbinden, umfasst.

8. Drehwellen-Pumpvorrichtung nach Anspruch 7, **dadurch gekennzeichnet, dass** diese eine oszillierende und rotierende Untereinheit (101) zum Pumpen eines Fluids nach Anspruch 5 umfasst, dass eine der Leitungen (11, 12) mit dem Einlass eines ersten Fluids verbunden ist, dass eine der Leitungen (111, 112) mit dem Einlass eines zweiten Fluids verbunden ist, dass die oszillierende und rotierende Vorrichtung zum Pumpen Mittel zum Mischen des ersten Fluids, das von der anderen der Leitungen (12, 11) zugeführt wird, und des zweiten Fluids, das von der anderen der Leitungen (112, 111) zugeführt wird, umfasst, wobei das erhaltene Gemisch proportional zur Differenz der Querschnitte zwischen den Arbeitskammern (31; 131) ist.

## Claims

1. A rotary reciprocating sub-assembly (1; 101) for pumping a fluid, in particular for medical use, said sub-assembly comprising: a hollow body (2; 102) defining a cavity (10; 110) of longitudinal axis (A) and having a wall with two ducts (11, 12; 111, 112) passing therethrough and opening out radially into said cavity (10; 110); and a piston (3; 103) housed in said cavity (10; 110) with which it co-operates to define a working chamber (31; 131), said piston (3; 103) being movable angularly between fluid-flow configurations in which it allows fluid-flow communication between said working chamber (31; 131) and one of said ducts (11, 12; 111, 112), and switching configurations in which it prevents any fluid-flow communication with each of said ducts, said piston (3; 103) being movable in reciprocating longitudinal translation so as to cause the dimensions of said working chamber (31; 131) to vary and successively suck in and then discharge said fluid via one and then the other of said ducts (11, 12; 111, 112), said sub-assembly being **characterized in that** said ducts (11, 12; 111, 112) are longitudinally offset from each other, **in that** said piston (3; 103) includes a channel (25; 125) that opens out longitudinally into said working chamber (31; 131) and radially into at least one pair of side recesses (22, 23; 122, 123) that are provided in the periphery of said piston (3; 103), that are longitudinally offset from each other, and that are arranged so that, in each fluid-flow configuration, only one of said recesses (22, 23; 122, 123) is in fluid-flow communication with only one of said ducts (11, 12; 111, 112), **in that** said recesses (22, 23; 122, 123) of said pair are angularly offset from each other by an angle that is different from 180°, and **in that** said ducts (11, 12; 111, 112) are angularly offset from each other by the same angle.

2. A rotary reciprocating pumping sub-assembly (1; 101) according to claim 1, **characterized in that** said recesses (22, 23; 122, 123) are superposed in a longitudinal plane, and **in that** said ducts (11, 12; 111, 112) are superposed in a longitudinal plane.

3. A rotary reciprocating pumping sub-assembly according to claim 1, **characterized in that** said piston includes n pairs of recesses distributed in two disjoint radial planes, and **in that** said channel is arranged to open out radially into said n recesses such that during one complete revolution of said piston in said body, said piston is n times in each of said fluid-flow configurations.

4. A rotary reciprocating pumping sub-assembly (101) according to claim 1, **characterized in that** it includes at least first and second levels, each corresponding, in distinct manner, to a set of two ducts (11, 12, 111, 112), to a working chamber (31; 131), to a channel (25; 125), and to a pair of recesses (22, 23; 122, 123).

5. A rotary reciprocating pumping sub-assembly (101) according to claim 5, **characterized in that** said working chambers (31; 131) present sections that are different.

6. A rotary reciprocating pumping sub-assembly (1; 101) according to claim 1, **characterized in that** it includes at least a cam and a guide finger (9), one carried by said piston (3; 103), the other by said body (2; 102), and arranged to co-operate reciprocally so that turning said piston (3; 103) relative to said body (2; 102) causes:
• over a first angular portion, said piston (3; 103) to move in axial translation (T1) relative to said body (2; 102) in a first direction;
• over a second angular portion, said piston (3; 103) to be axially stationary relative to said body (2; 102);
• over a third angular portion, said piston (3; 103) to move in axial translation relative to said body (2; 102) in a second direction; and
• over a fourth angular portion, said piston (3; 103) to be axially stationary relative to said body (2; 102);
said ducts (11, 12; 111, 112) and said recesses (22, 23; 122, 123) being arranged so that said ducts (11, 12; 111, 112) are closed during said second and fourth angular portions.

7. A rotary reciprocating fluid pumping device, in particular for medical use, the device being **characterized in that** it comprises drive means and a rotary reciprocating sub-assembly (1; 101) for pumping a fluid according to any preceding claim, and releasable mechanical coupler means for mechanically connecting said drive means to said piston (3; 103) in releasable manner.

8. A rotary reciprocating pumping device according to claim 7, **characterized in that** it includes a rotary reciprocating sub-assembly (101) for pumping a fluid according to claim 5, **in that** one of said ducts (11, 12) is connected to the inlet for a first fluid, one of said ducts (111, 112) is connected to the inlet for a second fluid, **in that** said rotary reciprocating pumping device includes mixer means for mixing said first fluid discharged by the other of said ducts (12, 11), and said second fluid discharged by the other of said ducts (112, 111), the resulting mixture being proportional to the difference in section between said working chambers (31; 131).
